# EUROPÄISCHE PATENTSCHRIFT

(11) **EP 3 717 095 B1**
(45) Veröffentlichungstag und Bekanntmachung des Hinweises auf die Patenterteilung: **26.07.2023**
(21) Anmeldenummer: 18786742.9
(22) Anmeldetag: 11.10.2018
(51) Int. Cl.: B01D 29/05, B01D 29/085, B01D 63/08

(54) **ANSCHLUSSEINRICHTUNG FUER EINE ABSAUGVORRICHTUNG EINES VAKUUMMEMBRANFILTERS**
CONNECTION DEVICE FOR A SUCTION APPARATUS OF A VACUUM DIAPHRAGM FILTER
SYSTÈME DE RACCORDEMENT DESTINÉ À UN DISPOSITIF D'ASPIRATION D'UN FILTRE À MEMBRANE SOUS VIDE

(30) Priorität: 27.11.2017 DE 102017127969
(43) Veröffentlichungstag der Anmeldung: 07.10.2020
(73) Patentinhaber: Sartorius Stedim Biotech GmbH, 37079 Göttingen (DE)
(72) Erfinder: PFLANZ, Karl, 37079 Goettingen (DE); PRUEHL, Sebastian, 37079 Goettingen (DE); SCHUETZLER, Michael, 37079 Goettingen (DE); GROSSMANN, Juliane, 37079 Goettingen (DE)
(74) Vertreter: Vigand, Philippe
(86) Internationale Anmeldenummer: PCT/EP2018/077789
(87) Internationale Veröffentlichungsnummer: WO 2019/101428

(56) Entgegenhaltungen:
- WO-A2-2015/032464
- US-A- 5 375 477
- US-A1- 2007 144 959

## Beschreibung

Die Erfindung betrifft eine Anschlusseinrichtung für eine Absaugvorrichtung für Vakuum-Membranfiltrationsanwendungen.

Im Rahmen mikrobiologisch-hygienischer Untersuchungen von wässrigen Proben wird üblicherweise die sogenannte Membranfiltermethode angewandt, bei der ein Membranfilter mit passender Porengröße in ein Filtrationsgerät eingelegt und die Probe filtriert wird, sodass die im Untersuchungsmaterial enthaltenen Mikroorganismen durch die Rückhaltewirkung des Membranfilters auf der Filteroberfläche zurückgehalten werden. Die in flüssigen Medien vorliegenden Kontaminationen sind in der Regel nur gering, weshalb die Prüfung typischerweise Probemengen von mehr als 100 ml erfordert.

In bekannten Vakuum-Membranfiltrationsvorrichtungen für die mikrobiologische Untersuchung flüssiger Medien besteht die Filtrationsvorrichtung aus einer Filtrationsbasis, einem Membranfilter (bevorzugt 47 bis 50 mm Durchmesser) und einem Trichter zum Aufgießen der flüssigen Probe. In den meisten Anwendungen werden die Filtrationsvorrichtungen auf wiederverwendbare Edelstahl-Absaugleisten montiert, über die ein Unterdruck (nachfolgend der Einfachheit halber allgemein als Vakuum bezeichnet) das Filtrat zur Pumpe abführt. Zudem wird ein Nährmedium benötigt, mit dem das Filter nach erfolgter Filtration in Kontakt gebracht wird. Typisch sind hierfür Petrischalen in unterschiedlichen Größen (bevorzugt ca. 55 mm Durchmesser).

Während Absaugleiste, Filtrationsbasis und Membranfilter für diese Probemengen unverändert bleiben, wird die Aufgusstrichtergröße meist dem Volumen des zu untersuchenden Mediums angepasst. Deshalb sind Systeme vorteilhaft, bei denen unterschiedliche Versionen des Trichters für dieselbe Aufnahme bzw. Filtrationsbasis geeignet sind.

Filtrationsbasis und Aufgusstrichter sind als Edelstahleinheiten, sowie in diversen Konstruktionsformen auch aus Kunststoff erhältlich. In letzterem Fall sind diese meist vorsterilisiert erhältlich und für die Einwegverwendung gedacht. Bei allen Handhabungsschritten steht das Bewahren der Sterilität der verwendeten Hilfsmittel im Vordergrund, da ansonsten das Produkt und/oder das diagnostische Ergebnis verfälscht würden.

Bei Edelstahlausführungen muss der Membranfilter grundsätzlich separat zwischen Aufgusstrichter und Filtrationsbasis eingefügt werden. Um Sterilität zu gewährleisten, wird das Einfügen typischerweise mit Hilfe einer hitzesterilisierten Pinzette vorgenommen. Die Entnahme des Membranfilters für nachfolgende Untersuchungen erfolgt wiederum mit einer erneut hitzesterilisierten Pinzette.

Daneben gibt es diverse Membranfiltrationsvorrichtungen aus Kunststoff, in denen der Membranfilter schon eingefügt ist und die komplette Vorrichtung steril zur Verfügung gestellt wird. Hier entfällt die Aufgabe des Einfügens des Membranfilters in die Filtrationsvorrichtung.

Wie bereits erwähnt, werden in den meisten Anwendungen die Filtrationsvorrichtungen auf wiederverwendbare Edelstahl-Absaugleisten montiert, über die das Vakuum das Filtrat zur Pumpe abführt. Da die von dem Membranfilter abgewandte Seite direkt mit der Vakuumquelle und anderen Leitungen und Behältnissen verbunden ist, werden diese Bereiche als unsteril betrachtet. In der Regel wird die der Filtration abgewandte Seite auch nicht vor jeder Filtration desinfiziert. Dies geschieht meist erst am Tagesende, z. B. über Nacht.

Während der Vakuum-Membranfiltration liegt auf der von der Filtration abgewandten Seite ein Vakuum an, das auch nach Abschluss der Filtration nicht gebrochen wird. Solange keine Belüftung zwischen Membranfilter und Filtrat erfolgt, kann die Restflüssigkeit nicht ablaufen. Die Belüftung wird jedoch durch den benetzten Membranfilter blockiert.

Eine Abnahme des benetzten Membranfilters bei anliegendem Vakuum ist erschwert, weshalb in unterschiedlichen auf dem Markt befindlichen Systemen das Vakuum über die Absaugleiste oder die Pumpeneinheit gebrochen wird. Da direkt unter der Filtrationsbasis aber ein Vakuum-Hohlraum anliegt, kann es zu einem Hochspritzen der nicht abgelaufenen Filtratrestmenge kommen. Bei diesem Vorgang werden Rückkontaminationen durch aufspritzendes Wasser erzeugt.

Unter dem Produktnamen Combisart^{®} sind von der Anmelderin vertriebene Vakuum-Membranfiltrationssysteme mit einer Edelstahl-Absaugleiste bekannt, die Anschlussmöglichkeiten für mehrere Filterstationen bereitstellt. Jede der Filterstationen kann über den jeweiligen Anschluss der Absaugleiste steril belüftet werden, um während der Belüftung eine Sekundärkontamination der Unterseite des Filters auszuschließen. Mit einem Bedienelement kann zwischen einer Vakuumstellung und einer Belüftungsstellung gewechselt werden. In der Vakuumstellung ist ein Saugkanal freigegeben, der zentral unter dem waagerecht angeordneten Membranfilter mündet. Über den Saugkanal wird die in der jeweiligen Filterstation enthaltene flüssige Substanz durch den Membranfilter angesaugt. In der Belüftungsstellung sperrt ein Schließelement den Saugkanal und gibt gleichzeitig einen Belüftungskanal frei, indem das Schließelement eine Strömungsverbindung zwischen einem separaten Belüftungsdurchgang und dem oberen Teil des Saugkanals herstellt. Der Belüftungskanal ist somit durch den Belüftungsdurchgang und den oberen Teil des Saugkanals gebildet, der zentral unter demr Membranfilter mündet.

Des Weiteren sind z. B. aus der EP 1 556 152 B1 und der CN 102586082 A Unterdruck-Drainageeinrichtungen für Filtrationsvorrichtungen mit einem Saugkanal bekannt, der auf einer dem Membranfilter zugewandten Aufnahmefläche eines mechanischen Trägers mündet, um die in der Filtrationsvorrichtung enthaltene flüssige Substanz durch den Membranfilter anzusaugen. Zur Belüftung ist hier jeweils ein vom Saugkanal vollständig getrennter Belüftungskanal vorgesehen, der mit einem eigenen Ventil verschlossen und geöffnet werden kann.

Die US 2007/0144959 A1 zeigt ein Filtrationssystem mit einer Filtrationsvorrichtung, die in einer Basis gehalten ist. Die Filtrationsvorrichtung umfasst einen Adapter und einen auf dessen Oberseite angeordneten Aufnahmebehälter. Der Aufnahmebehälter enthält ein Filterelement und nimmt eine zu filternde Flüssigkeit auf. Ein Ausgabegefäß ist über ein Gewinde lösbar auf der Unterseite des Adapters befestigt. In der Basis ist ein interner Vakuumkanal gebildet, über den ein Vakuum im Ausgangsbehälter erzeugt werden kann. Eine Bohrung in der Basis dient zur Aufnahme eines Dreiwegeventils und weist einen Entlüftungsschlitz auf. In der geöffneten Stellung des Ventils verbindet dessen Vakuumanschluss den oberen und unteren Teil des Vakuumkanals, und der Entlüftungsanschluss des Ventils ist durch die Seitenwand der Bohrung, die den Entlüftungsschlitz nicht enthält, abgesperrt. In der geschlossenen Stellung, stehen der Entlüftungsanschluss und der Vakuumanschluss in Fluidverbindung mit dem Entlüftungsschlitz.

Die WO 2015/032464 A2 zeigt eine Filtrationsvorrichtung mit einer Anschlusseinrichtung gemäß den Merkmalen des Oberbegriffs des Anspruchs 1. Die Filtrationseinrichtung umfasst mindestens zwei, vorzugsweise identische, Filtertrichter, die jeweils einen Einlass, einen Auslass und einen Innenraum aufweisen, der zur Aufnahme eines gewünschten Volumens des zu filtrierenden Probenfluids dimensioniert ist. Die Filtertrichter sind mit Hilfe eines Filterhalters lösbar miteinander verbunden. Der erste Filtertrichter ist mit einer Anschlussstruktur versehen, um eine Gegenanschlussstruktur des Filterhalters lösbar und mechanisch abdichtend mit dem ersten Filtertrichter zu verbinden, so dass die gesamte im Innenraum des ersten Filtertrichters befindliche Flüssigkeit das erste Filtermedium ohne Bypass passieren muss. Der Filterhalter hat eine zweite Anschlussstruktur zur lösbaren und mechanisch dichtenden Befestigung an einer passenden Anschlussstruktur eines Zwischenverbindungsstücks, so dass das gesamte Fluid, das das erste Filtermedium passiert, über das Zwischenverbindungsstück zum Einlass des zweiten Filtertrichters geleitet wird. Das Zwischenverbindungsstück hat einen Anschluss an einer Seitenwand, der über eine Verrohrung mit einer externen Ventilanordnung verbunden ist. Die Ventilanordnung umfasst ein Drei-WegeVentil, das zwischen verschiedenen Positionen umgeschaltet werden kann, um eine selektive Strömungsverbindung des Innenraums des zweiten Filtertrichters über den Anschluss mit einer externen Gasquelle oder der Umgebung oder einer Vakuumquelle zu ermöglichen.

Aufgabe der vorliegenden Erfindung ist es, die zur Vermeidung von Rücckontaminationen erforderliche Belüftung des Bereichs unterhalb des Membranfilters zu vereinfachen, insbesondere durch eine Anschlusseinrichtung, die ohne komplizierte Ventile oder elektrische Aktuatoren auskommt und mit wenigen einfach montier- und demontierbaren, einfach zu reinigenden und sterilisierbaren Geräteteilen realisierbar ist.

Gelöst wird diese Aufgabe durch eine Anschlusseinrichtung für eine Absaugvorrichtung für Vakuum-Membranfiltrationsanwendungen mit den Merkmalen des Anspruchs 1. Vorteilhafte und zweckmäßige Ausgestaltungen der erfindungsgemäßen Anschlusseinrichtung sind in den Unteransprüchen angegeben.

Die erfindungsgemäße Anschlusseinrichtung für eine Absaugvorrichtung ist für Vakuum-Membranfiltrationsanwendungen vorgesehen und umfasst einen Träger zur Aufnahme eines Membranfilters oder einer Filtrationsbasis, einen unterhalb des Membranfilters bzw. der Filtrationsbasis gebildeten Hohlraum, einen Saugkanal, der zentral in den Hohlraum mündet, und einen Belüftungskanal, der seitlich in den Hohlraum mündet. Gemäß der Erfindung umfasst die Anschlusseinrichtung ferner ein in mehrere Schaltstellungen bewegbares Schließelement, das in Abhängigkeit der Schaltstellungen sowohl den Saugkanal als auch den Belüftungskanal sperren oder freigeben kann.

Die Erfindung beruht auf der Erkenntnis, dass bei einer Anschlusseinrichtung zusätzliche Bauteile eingespart werden können, wenn das Schließelement so ausgebildet ist, dass es alleine, also unabhängig von weiteren Einstellorganen, in den jeweiligen Schaltstellungen nicht nur den Saugkanal, sondern gleichzeitig auch den Belüftungskanal freigeben oder sperren kann. Auf ein zusätzliches Ventil oder dergleichen zur selektiven Freigabe des Belüftungskanals kann deshalb verzichtet werden. Das in die verschiedenen Schaltstellungen bewegbare Schließelement lässt sich so gestalten, dass bei einem Wechsel der Schaltstellung alleine durch die Bewegung des Schließelements der Saugkanal und gleichzeitig der Belüftungskanal in den der Schaltstellung entsprechenden Zustand (freigegeben oder gesperrt) versetzt wird. Dank des Entfalls eines zusätzlichen Einstellorgans für den Belüftungskanal vereinfacht sich der Aufbau der erfindungsgemäßen Anschlusseinrichtung, die dementsprechend kostengünstig in der Herstellung ist.

Die erfindungsgemäße Anschlusseinrichtung ist in der Lage, bei gewünschter Belüftung das am Saugkanal anliegende Vakuum unterhalb des Membranfilters zu reduzieren bzw. einen Druckausgleich zur Umgebung zu schaffen, sodass es nicht zu einem Rückspritzen des Filtrats an die Unterseite des Membranfilters kommen kann und gleichzeitig zurückgebliebene Flüssigkeit unter dem Membranfilter abgesaugt wird.

Der Belüftungskanal sollte möglichst weit oben in den Hohlraum unter dem Membranfilter münden, um möglichst effektiv zu verhindern, dass im Hohlraum befindliche oder hochspritzende Flüssigkeit in den Belüftungskanal gelangen kann. Dementsprechend mündet bei einer bevorzugten Ausführungsform der Erfindung der Belüftungskanal über einem Sammelabschnitt des Hohlraums. Unter einem Sammelabschnitt soll hier derjenige untere Bereich des Hohlraums unter dem Membranfilter verstanden werden, in dem sich während oder nach Beendigung eines Filtrationsvorgangs (Rest-)Flüssigkeit befinden kann. Die Einmündung des Belüftungsabschnitts liegt also höher als der Flüssigkeitsspiegel der (Rest-)Flüssigkeit, wie er typischerweise während oder nach einer Filtration vorzufinden ist.

Bei der erfindungsgemäßen Anschlusseinrichtung ist der Belüftungskanal in jeder Schaltstellung vom Saugkanal getrennt, d. h. es besteht nie eine direkte Strömungsverbindung zwischen dem Belüftungskanal und dem Saugkanal - anders als beispielsweise bei den Combisart^{®}-Vakuum-Membranfiltrationssystemen. Die durchgehende Trennung erlaubt es, den Saugkanal und den Belüftungskanal nach den jeweiligen Anforderungen zu gestalten, ohne dass hierbei Kompromisse eingegangen werden müssen. Der entscheidende Vorteil besteht jedoch darin, dass der Saugkanal und der Druckkanal unabhängig voneinander durch das Schließelement freigegeben oder gesperrt werden können. Dies ermöglicht insbesondere die zum Druckausgleich unter dem Membranfilter bei gleichzeitigem Absaugen der Restflüssigkeit gewünschte Schaltstellung, in der sowohl der Saugkanal als auch der Druckkanal freigegeben sind.

In einer bevorzugten Ausführungsform der Erfindung weist das Schließelement sowohl wenigstens einen Saugkanalabschnitt zur Vervollständigung des Saugkanals in wenigstens einer Schaltstellung als auch wenigstens einen Belüftungskanalabschnitt zur Vervollständigung des Belüftungskanals in wenigstens einer Schaltstellung auf. Durch geeignete Bewegung des Schaltelements lassen sich diese Kanalabschnitte so in Position bringen, dass der Saugkanal oder der Belüftungskanal oder beide vervollständigt und damit freigegeben sind.

Die erfindungsgemäße Anschlusseinrichtung hat wenigstens zwei, im Idealfall drei verschiedene Schaltstellungen:

In einer ersten Schaltstellung ist der Saugkanal freigegeben und der Belüftungskanal gesperrt. Dies entspricht einer "Vakuum"-Stellung, in der ein Filtrationsbetrieb stattfindet und keine gleichzeitige Belüftung gewünscht ist.

In einer zweiten Schaltstellung sind sowohl der Saugkanal als auch der Belüftungskanal freigegeben. Dies entspricht einer "Belüftung"-Stellung, in der nach Abschluss einer Filtration das am Saugkanal weiterhin anliegende Vakuum dafür sorgt, dass die Restflüssigkeit abgesaugt wird, ohne dass es zu einem Hochspritzen kommt.

In einer dritten Schaltstellung ist der Saugkanal gesperrt und der Belüftungskanal freigegeben. Dies entspricht einer "Stop"-Stellung, in der kein Filtrationsbetrieb vorgesehen, aber eine Belüftung sinnvoll bzw. erwünscht ist, um das Abnehmen des Membranfilters bzw. der Filtrationsbasis vom Träger zu erleichtern.

Die oben angegebenen Schaltstellungen lassen sich in Bezug auf den Saugkanal am einfachsten dadurch erreichen, dass im Schließelement zwei Saugkanalabschnitte gebildet sind, von denen einer in der ersten Schaltstellung ("Vakuum") und der andere in der zweiten Schaltstellung ("Belüftung") den Saugkanal vervollständigt.

In Bezug auf den Belüftungskanal lassen sich die Schaltstellungen am einfachsten dadurch erreichen, dass im Schließelement zwei Belüftungskanalabschnitte gebildet sind, von denen einer in der zweiten Schaltstellung ("Belüftung") und der andere in der dritten Schaltstellung ("Stop") den Belüftungskanal vervollständigt.

Besonders bevorzugt ist eine Ausführungsform der Erfindung, bei der das Schließelement drehbar in einem Körper der Anschlusseinrichtung gelagert ist, in den auch der Träger eingesetzt ist. Eine Drehung des Schließelements in die verschiedenen Schaltstellungen, gegebenenfalls mithilfe eines am Schließelement angreifenden Bedienelements, ist eine für den Bediener leicht und intuitiv durchzuführende Schaltbewegung. Neben der drehbaren Lagerung des Schließelements erfüllt der Körper bei dieser Ausführungsform aber auch noch eine weitere Funktion, indem er als Aufnahme für den Träger der Anschlusseinrichtung dient.

Der Körper der Anschlusseinrichtung, in den der Träger eingesetzt ist, ist bevorzugt aus Edelstahl oder Aluminium gefertigt und fest oder lösbar mit einer Absaugvorrichtung verbunden, die typischerweise ebenfalls aus Edelstahl oder Aluminium gefertigt ist.

Das Schließelement ist vorzugsweise lösbar im Körper gelagert. Da das Schließelement aufgrund seiner Beweglichkeit und des Mediumkontakts verschleißen kann, ist es zweckmäßig, dieses als Austauschteil zu konzipieren.

Beim oben geschilderten Aufbau der Anschlusseinrichtung mit einem Körper, in dem das Schließelement drehbar gelagert ist, und einem in den Körper fest, aber lösbar, eingesetzten Träger ist es notwendig, dass der Belüftungskanal einen Belüftungskanalabschnitt im Körper und einen Belüftungskanalabschnitt im Träger aufweist. Körper und Träger sind typischerweise im Wesentlichen rotationssymmetrisch ausgebildet. Um zu vermeiden, dass der Träger zwingend in einer bestimmten Drehstellung relativ zum Körper eingesetzt sein muss, damit die Belüftungskanalabschnitte miteinander fluchten, ist bei einer bevorzugten Ausführungsform ein sich im Wesentlichen ringförmig zwischen dem Körper und dem Träger gebildeter, abgedichteten Hohlraum vorgesehen. Dieser z. B. mittels O-Ringen abgedichtete Hohlraum sorgt in jedem Fall für eine Strömungsverbindung zwischen dem Belüftungskanalabschnitt des Körpers und dem Belüftungskanalabschnitt des Trägers, unabhängig von der Drehstellung.

Insbesondere im Hinblick auf eine einfache Bedienung ist eine Ausführung der Anschlusseinrichtung als Absperrhahn mit einem Schließelement in Form eines Kükens (konusförmiger Absperrkörper) zweckmäßig.

Die verschiedenen Schaltstellungen werden erfindungsgemäß durch Bewegen des Schließelements in entsprechende Stellungen erreicht. Deshalb ist es vorteilhaft, das Schließelement mechanisch an ein Bedienelement zu koppeln, sodass ein Umschalten zwischen den Schaltstellungen für den Anwender problemlos möglich ist.

Vorzugsweise ist die erfindungsgemäße Anschlusseinrichtung mit einem Rastmechanismus für das Schließelement versehen, der das Schließelement definiert in der jeweiligen Schaltstellung hält.

Um eine Kontamination der Unterseite des Membranfilters während der Belüftung auszuschließen, sollte dem Belüftungskanal der Anschlusseinrichtung ein Luft-Sterilfilter vorgeschaltet sein.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figuren 1a und 1b eine seitliche Außenansicht und eine um 90° gedrehte seitliche Schnittansicht einer erfindungsgemäßen Anschlusseinrichtung für eine Absaugvorrichtung in einer ersten Schaltstellung ("Vakuum");
- Figuren 2a und 2b eine seitliche Außenansicht und eine um 90° gedrehte seitliche Schnittansicht der erfindungsgemäßen Anschlusseinrichtung in einer zweiten Schaltstellung ("Belüftung"); und
- Figuren 3a und 3b eine seitliche Außenansicht und eine um 90° gedrehte seitliche Schnittansicht der erfindungsgemäßen Anschlusseinrichtung in einer dritten Schaltstellung ("Stop").

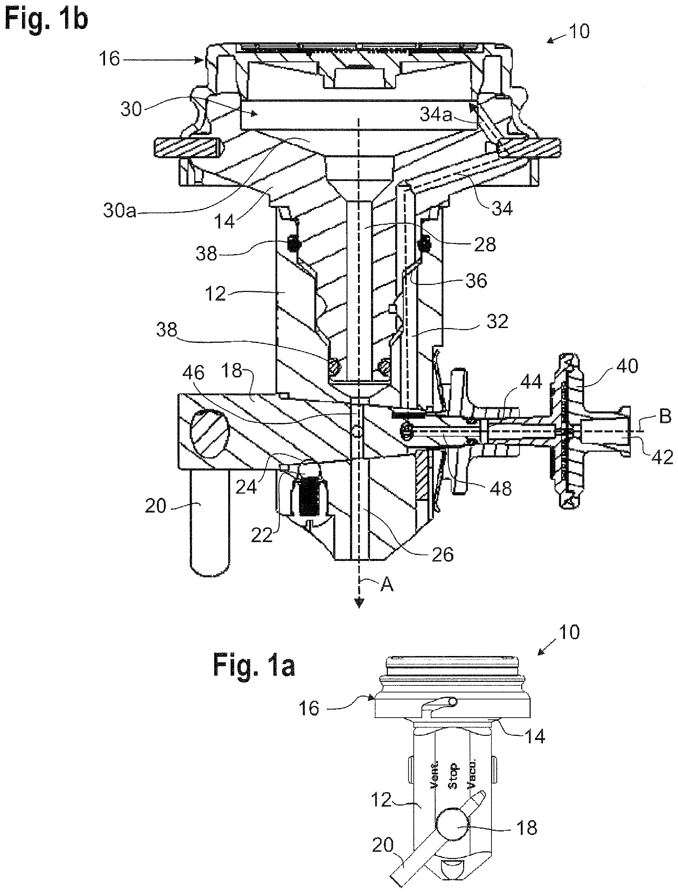

In den Figuren 1a und 1b ist eine Anschlusseinrichtung 10 aus Edelstahl für eine Absaugvorrichtung dargestellt, die für Vakuum-Membranfiltrationsanwendungen vorgesehen ist.

Die Anschlusseinrichtung 10 umfasst einen länglichen Körper 12, in den ein Träger 14 eingesetzt ist. Der Träger 14 ist im eingesetzten Zustand fest und dicht mit dem Körper 12 verbunden, kann aber aus dem Körper 12 auch wieder herausgenommen werden. Dies ist im Hinblick auf die Reinigung der Anschlusseinrichtung 10 und die Möglichkeit, verschiedene Träger 14 in den Körper 12 einzusetzen, von Bedeutung.

Auf den Träger 14 ist eine Filtrationsbasis 16 aufgesetzt. Für eine Vakuum-Membranfiltration wird auf der Filtrationsbasis 16 ein Aufgusstrichter montiert, wobei zwischen der Filtrationsbasis 16 und dem Trichter dann ein Membranfilter eingeklemmt wird.

Unterhalb des Trägers 14 ist im Körper 12 ein Schließelement 18 drehbar gelagert, wobei die Längsachsen des Körpers 12 und des Trägers 14 konzentrisch und senkrecht zur waagerechten Drehachse des Schließelements 18 verlaufen. Das Schließelement 18 ist als kegelstumpfförmiges Küken ausgebildet und Teil eines Absperrhahns. Im Schließelement 18 sind verschiedene Kanalabschnitte gebildet, die später bei der Erläuterung der Funktionsweise noch genauer beschrieben werden. Zum Absperrhahn gehört noch ein außerhalb des Körpers 12 am Schließelement 18 angreifendes Bedienelement 20 zur manuellen Betätigung des Schließelements 18.

Das Schließelement 18 kann mithilfe des Bedienelements 20 in mindestens zwei, beim dargestellten Ausführungsbeispiel in drei verschiedene stabile Schaltstellungen gedreht werden. Hierfür ist ein Rastmechanismus mit einem im Körper 12 angeordneten und in Richtung des Schließelements 18 vorgespannten Rastelement 22 vorgesehen. Das Rastelement 22 kann in bekannter Weise in einzelne Rastausnehmungen 24 eingreifen, die im Schließelement 18 gebildet sind und die einzelnen Schaltstellungen definieren.

Im Körper 12 und im Träger 14 sind zu beiden Seiten des Schließelements 18 zentrale Saugkanalabschnitte 26 bzw. 28 ausgebildet, die die wesentlichen Teile eines Saugkanals bilden. Der erste Saugkanalabschnitt 26 im Körper 12 mündet auf der vom Schließelement 18 abgewandten Seite in einen Absaugkanal einer nicht gezeigten Edelstahl-Absaugleiste. Der zweite Saugkanalabschnitt 28 im Träger 14 mündet auf der vom Schließelement 18 abgewandten Seite zentral in einen Hohlraum 30, der sich unterhalb der einzusetzenden Filtrationsbasis 16 befindet. Ein unterer Abschnitt dieses Hohlraums 30, in dem sich während oder nach Beendigung eines Filtrationsvorgangs (Rest-)Flüssigkeit befinden kann, wird als Sammelabschnitt 30a bezeichnet.

Unabhängig von den Saugkanalabschnitten 26, 28 sind im Körper 12 und im Träger 14 Belüftungskanalabschnitte 32 bzw. 34 gebildet, die in Strömungsverbindung miteinander stehen und Teile eines Belüftungskanals bilden. Die Strömungsverbindung zwischen den beiden Belüftungskanalabschnitten 32 und 34 kann dadurch hergestellt sein, dass diese einander gegenüberliegende miteinander fluchtende Öffnungen aufweisen, was jedoch eine bestimmte Drehstellung des Trägers 14 relativ zum Körper 12 voraussetzt. Im dargestellten Ausführungsbeispiel ist deshalb ein im Wesentlichen ringförmig umlaufender Hohlraum 36 zwischen dem Körper 12 und dem Träger 14 vorgesehen, in den jeweils eine Öffnung des im Körper 12 gebildeten Belüftungskanalabschnitts 32 und eine

Öffnung des im Träger 14 gebildeten Belüftungskanalabschnitts 34 mündet. Der Hohlraum 36 ist abgedichtet, beim dargestellten Ausführungsbeispiel durch zwei auf unterschiedlichen axialen Höhen zwischen dem Körper 12 und dem Träger 14 angeordneten O-Ringen 38. Dank des umlaufenden Hohlraums 36 ist die Strömungsverbindung zwischen den beiden Belüftungskanalabschnitten 32 und 34 unabhängig von der Drehstellung des Trägers 14 relativ zum Körper 12 immer gewährleistet.

Das vom unteren Belüftungskanalabschnitt 32 abgewandte Ende des oberen Belüftungskanalabschnitts 34 im Träger 14 mündet seitlich in den Hohlraum 30 unter der einzusetzenden Filtrationsbasis 16. Die Einmündung 34a ist oberhalb des Sammelabschnitts 30a des Hohlraums 30 auf einer axialen Höhe angeordnet, die - unter normalen Umständen - über dem Flüssigkeitsspiegel einer während oder nach der Filtration im Hohlraum 30 vorhandenen (Rest-)Flüssigkeit liegt.

Der untere Belüftungskanalabschnitt 32 im Körper 12 führt zur Mantelfläche des Schließelements 18.

Zum Belüftungskanal gehört noch ein - hier in Verlängerung der Drehachse des Schließelements 18 - seitlich am Körper 12 angeordneter Luft-Sterilfilter 40. Der Luft-Sterilfilter 40 hat einen Eingang 42, der mit der Umgebung in Kontakt ist, und einen Ausgang 44, der der vom Bedienelement 20 abgewandten Stirnfläche des Schließelements 18 zugewandt ist.

Der Luft-Sterilfilter 40 kann gemäß einer nicht gezeigten alternativen Ausführungsform auch an einer anderen Stelle seitlich am Körper 12 angeordnet sein. In diesem Fall ist der Ausgang 44 des Luft-Sterilfilters 40 der Seitenwand des Körpers 12 zugewandt. Beispielsweise kann der Luft-Sterilfilters 40 bezogen auf die vertikale Mittelachse in der Darstellung der Figur 1b um 90° versetzt angeordnet sein, sodass der Strömungsweg durch den Luft-Sterilfilter 40 nicht in der Papierebene, sondern senkrecht zur Papierebene verliefe.

Nachfolgend wird die Funktionsweise der Anschlusseinrichtung 10 anhand der in den Figuren 1a, 1b und 2a, 2b sowie 3a, 3b gezeigten drei Schaltstellungen erläutert.

Die Figuren 1a und 1b zeigen die Anschlusseinrichtung 10 in einer ersten Schaltstellung, die nachfolgend als "Vakuum" bezeichnet wird. Die Schaltstellung "Vakuum" entspricht dem bestimmungsgemäßen Betrieb der Anschlusseinrichtung 10, wenn das in der eingesetzten Filtrationsbasis 16 enthaltene Medium durch den Membranfilter angesaugt werden soll. Dementsprechend ist der Saugkanal in dieser Schaltstellung freigegeben, was durch den nicht unterbrochenen Pfeil A symbolisiert ist, der die Saugrichtung angibt. Der Saugkanal ist in der Schaltstellung "Vakuum" freigegeben, weil ein dritter Saugkanalabschnitt 46 im Schließelement 18 den ersten Saugkanalabschnitt 26 im Körper 12 mit dem zweiten Saugkanalabschnitt 28 im Träger 14 verbindet.

Der Belüftungskanal ist dagegen in der "Vakuum"-Stellung durch das Schließelement 18 gesperrt. wie nachfolgend erläutert wird. Der dem Schließelement 18 zugewandte Ausgang 44 des Luft-Sterilfilters 40 mündet in einen waagerechten dritten Belüftungskanalabschnitt 48 im Schließelement 18. Der dritte Belüftungskanalabschnitt 48 verläuft entlang der Drehachse des Schließelements 18, sodass er unabhängig von der Drehstellung des Schließelements 18 immer in Strömungsverbindung mit dem Ausgang 44 des Luft-Sterilfilters 40 steht. In der "Vakuum"-Stellung steht aber kein im Schließelement 18 vom dritten Belüftungskanalabschnitt 48 abzweigender Belüftungskanalabschnitt mit dem zweiten Belüftungskanalabschnitt 32 im Körper 12 in Strömungsverbindung. Somit kann keine Luft aus der Umgebung in den Teil des Belüftungskanals gelangen, der in den Hohlraum 30 mündet. Dies ist durch den Querbalken im Schließelement 18 symbolisiert, der den Pfeil B unterbricht.

Im Falle der nicht gezeigten alternativen Anordnung des Luft-Sterilfilters 40 seitlich am Körper 12 ist der dritte Belüftungskanalabschnitt 48 nicht im Schließelement 18, sondern im Körper 12 gebildet, sodass der Ausgang 44 des Luft-Sterilfilters 40 in den Eingang des dritte Belüftungskanalabschnitts 48 mündet. In der "Vakuum"-Stellung steht aber, wie oben beschrieben, kein im Schließelement 18 gebildeter Belüftungskanalabschnitt mit dem zweiten Belüftungskanalabschnitt 32 im Körper 12 in Strömungsverbindung, sodass der Belüftungskanal gesperrt ist.

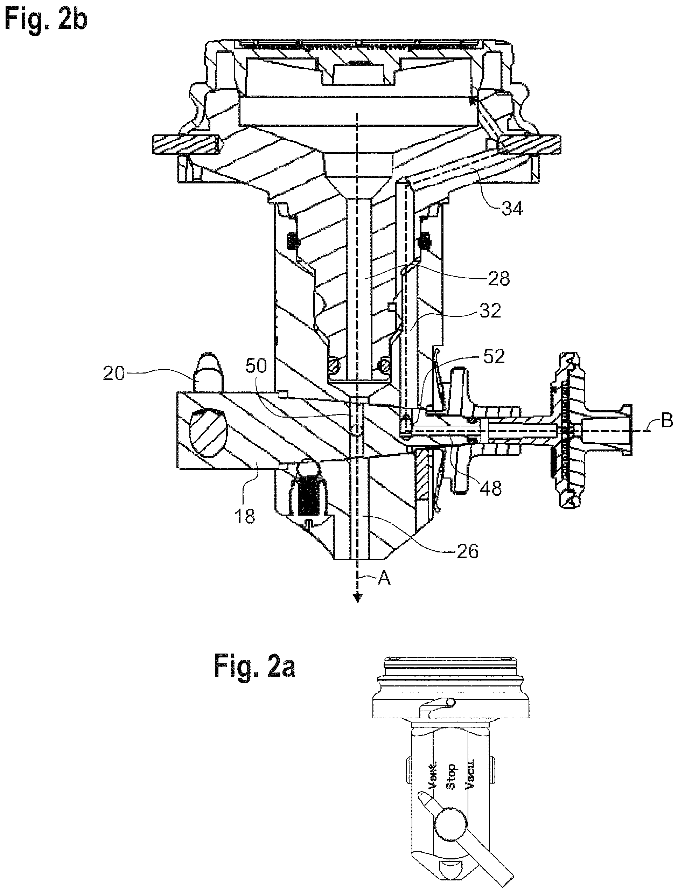

Ausgehend von der "Vakuum"-Stellung kann das Schließelement 18 mithilfe des Bedienelements 20 gegen den Uhrzeigersinn in die Schaltstellung "Belüftung" (engl.: "Vent.") gedreht werden, die in den Figuren 2a und 2b gezeigt ist. In dieser Schaltstellung sind sowohl der Saugkanal als auch der Belüftungskanal freigegeben, wie nachfolgend erläutert wird.

In der Schaltstellung "Belüftung" verbindet ein vierter Saugkanalabschnitt 50 im Schließelement 18 den ersten Saugkanalabschnitt 26 im Körper 12 mit dem zweiten Saugkanalabschnitt 28 im Träger 14, sodass der Saugkanal entsprechend dem nicht unterbrochenen Pfeil A freigegeben ist. (Der vierte Saugkanalabschnitt 50 ist in der Ansicht der Figur 1b aufgrund der anderen Drehstellung des Schließelements 18 nicht sichtbar.)

Gleichzeitig stellt im Schließelement 18 ein weiterer, vom dritten Belüftungskanalabschnitt 48 senkrecht abzweigender vierter Belüftungskanalabschnitt 52 eine Strömungsverbindung zwischen dem dritten Belüftungskanalabschnitt 48 und dem zweiten Belüftungskanalabschnitt 32 im Körper 12 her. (Der vierte Belüftungskanalabschnitt 52 ist in der Ansicht der Figur 1b aufgrund der anderen Drehstellung des Schließelements 18 nicht sichtbar.)

Im Fall der der nicht gezeigten alternativen Anordnung des Luft-Sterilfilters 40 seitlich am Körper 12 stellt der vierte Belüftungskanalabschnitt 52 im Schließelement 18 eine Strömungsverbindung zwischen dem dritten Belüftungskanalabschnitt 48 im Körper 12 und dem zweiten Belüftungskanalabschnitt 32 im Körper 12 her.

Somit kann entsprechend dem nicht unterbrochenen Pfeil B Luft aus der Umgebung nach Durchtritt durch den Luft-Sterilfilter 40 über die vier Belüftungskanalabschnitte 48, 52, 32, 34 seitlich in den Hohlraum 30 gelangen.

Die Schaltstellung "Belüftung" dient dazu, nach einer Filtration bei weiterhin anliegendem Vakuum ein sicheres Absaugen von Restflüssigkeit ohne Rückkontaminationsgefahr zu ermöglichen.

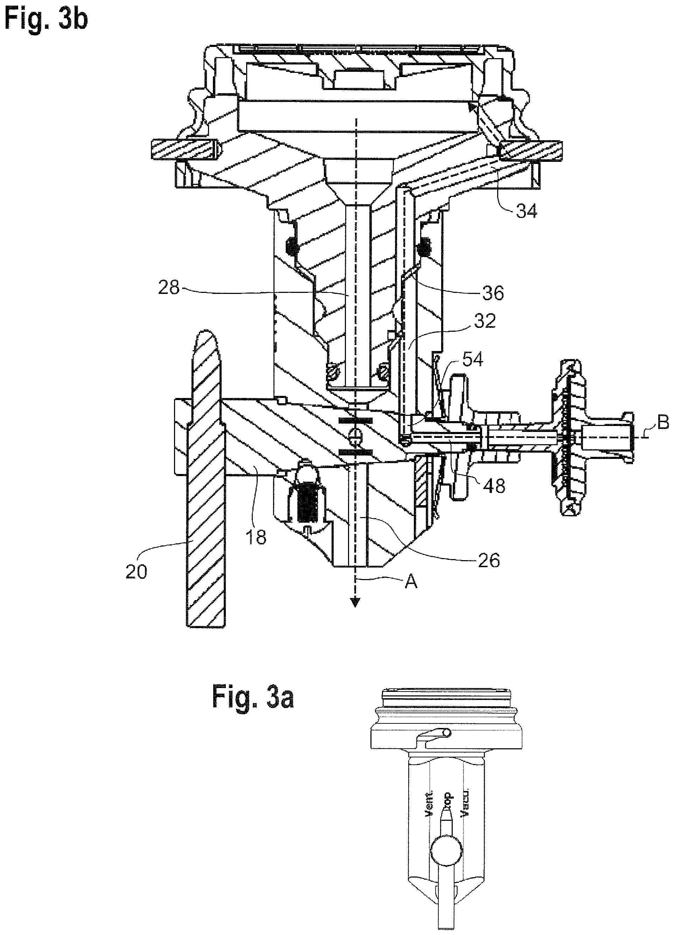

Um schließlich ein erleichtertes Abnehmen des benetzten Membranfilters zu ermöglichen, wird das Schließelement 18 mithilfe des Bedienelements 20 aus der Schaltstellung "Belüftung" im Uhrzeigersinn in die Stellung "Stop" gedreht. In dieser in den Figuren 3a und 3b gezeigten Schaltstellung ist der Saugkanal durch das Schließelement 18 unterbrochen, da weder der dritte Saugkanalabschnitt 46 noch der vierte Saugkanalabschnitt 50 eine Strömungsverbindung zwischen dem ersten Saugkanalabschnitt 26 im Körper 12 und dem zweiten Saugkanalabschnitt 28 im Träger 14 herstellt. Dies ist durch die beiden Querbalken symbolisiert, die den Pfeil A unterbrechen. Ein an der unteren Mündung des ersten Saugkanalabschnitts 26 noch anliegendes Vakuum der Absaugvorrichtung hat also keine Auswirkungen auf den Membranfilter auf der Filtrationsbasis 16.

In der Schaltstellung "Stop" ist aber weiterhin der Belüftungskanal freigegeben, da ein vom dritten Belüftungskanalabschnitt 48 im Schließelement 18 abzweigender fünfter Belüftungskanalabschnitt 54 in Strömungsverbindung mit dem zweiten Belüftungskanalabschnitt 32 im Körper 12 steht.

Im Fall der der nicht gezeigten alternativen Anordnung des Luft-Sterilfilters 40 seitlich am Körper 12 steht der im Körper 12 gebildete dritte Belüftungskanalabschnitt 48 über den fünften Belüftungskanalabschnitt 54 im Schließelement 18 in Strömungsverbindung mit dem zweiten Belüftungskanalabschnitt 32 im Körper 12.

Somit kann entsprechend dem nicht unterbrochenen Pfeil B Luft aus der Umgebung nach Durchtritt durch den Luft-Sterilfilter 40 über die vier Belüftungskanalabschnitte 48, 54, 32, 34 seitlich in den Hohlraum 30 gelangen.

Da somit zum einen die Belüftung unterhalb des Membranfilters gewährleistet ist und zum anderen ein noch anliegender Unterdruck der Absaugvorrichtung keine Anziehungskraft auf den Membranfilter ausüben kann, lässt sich der Membranfilter in der "Stop"-Stellung problemlos von der Filtrationsbasis 16 abheben.

Es ist zu beachten, dass die drei Schaltstellungen der Anschlusseinrichtung 10 alleine durch Drehen des Schließelements 18 erreicht werden, ohne dass ein zusätzliches Ventil oder dergleichen benötigt wird. Alle für die jeweilige Betriebsart benötigten Strömungsverbindungen bzw. -unterbrechungen im Saugkanal und im Belüftungskanal werden durch geeignet angeordnete Kanalabschnitte im drehbaren Schließelement 18 hergestellt.

### Bezugszeichenliste

- 10: Anschlusseinrichtung
- 12: Körper
- 14: Träger
- 16: Filtrationsbasis
- 18: Schließelement
- 20: Bedienelement
- 22: Rastelement
- 24: Rastausnehmungen
- 26: erster Saugkanalabschnitt im Körper
- 28: zweiter Saugkanalabschnitt im Träger
- 30: Hohlraum
- 30a: Sammelabschnitt des Hohlraums
- 32: zweiter Belüftungskanalabschnitt im Körper
- 34: erster Belüftungskanalabschnitt im Träger
- 34a: Einmündung des ersten Belüftungskanalabschnitts
- 36: Hohlraum
- 38: O-Ringe
- 40: Luft-Sterilfilter
- 42: Eingang des Luft-Sterilfilters
- 44: Ausgang des Luft-Sterilfilters
- 46: dritter Saugkanalabschnitt im Schließelement
- 48: dritter Belüftungskanalabschnitt im Schließelement bzw. Körper
- 50: vierter Saugkanalabschnitt im Schließelement
- 52: vierter Belüftungskanalabschnitt im Schließelement
- 54: fünfter Belüftungskanalabschnitt im Schließelement
- A: Saugrichtung
- B: Belüftungsrichtung

## Patentansprüche

1. Anschlusseinrichtung (10) für eine Absaugvorrichtung für Vakuum-Membranfiltrationsanwendungen, mit
einem Träger (14) zur Aufnahme eines Membranfilters oder einer Filtrationsbasis (16),
einem unterhalb des Membranfilters bzw. der Filtrationsbasis (16) gebildeten Hohlraum (30),
einem Saugkanal, der zentral in den Hohlraum (30) mündet, und
einem Belüftungskanal, der seitlich in den Hohlraum (30) mündet,
**dadurch gekennzeichnet,**
**dass** die Anschlusseinrichtung (10) ein in mehrere Schaltstellungen bewegbares Schließelement (18) umfasst, das in Abhängigkeit der Schaltstellungen sowohl den Saugkanal als auch den Belüftungskanal sperren oder freigeben kann, und
**dass** der Belüftungskanal in jeder Schaltstellung vom Saugkanal getrennt ist.

2. Anschlusseinrichtung (10) nach Anspruch 1, **dadurch gekennzeichnet, dass** der Belüftungskanal über einem Sammelabschnitt (30a) des Hohlraums (30) in den Hohlraum (30) mündet.

3. Anschlusseinrichtung (10) nach einem der vorhergehenden Ansprüche, **dadurch gekennzeichnet, dass** das Schließelement (18) sowohl wenigstens einen Saugkanalabschnitt (46, 50) zur Vervollständigung des Saugkanals in wenigstens einer Schaltstellung als auch wenigstens einen Belüftungskanalabschnitt (48, 52, 54) zur Vervollständigung des Belüftungskanals in wenigstens einer Schaltstellung aufweist.

4. Anschlusseinrichtung (10) nach einem der vorhergehenden Ansprüche, **dadurch gekennzeichnet, dass** in einer ersten Schaltstellung der Saugkanal freigegeben und der Belüftungskanal gesperrt ist.

5. Anschlusseinrichtung (10) nach einem der vorhergehenden Ansprüche, **dadurch gekennzeichnet, dass** in einer zweiten Schaltstellung sowohl der Saugkanal als auch der Belüftungskanal freigegeben sind.

6. Anschlusseinrichtung (10) nach einem der vorhergehenden Ansprüche, **dadurch gekennzeichnet, dass** in einer dritten Schaltstellung der Saugkanal gesperrt und der Belüftungskanal freigegeben ist.

7. Anschlusseinrichtung (10) nach den Ansprüchen 4 bis 6, **dadurch gekennzeichnet, dass** im Schließelement (18) zwei Saugkanalabschnitte (46, 50) gebildet sind, von denen einer (46) in der ersten Schaltstellung und der andere (50) in der zweiten Schaltstellung den Saugkanal vervollständigt.

8. Anschlusseinrichtung (10) nach den Ansprüchen 4 bis 6 oder Anspruch 7, **dadurch gekennzeichnet, dass** im Schließelement (18) zwei Belüftungskanalabschnitte (52, 54) gebildet sind, von denen einer (52) in der zweiten Schaltstellung und der andere (54) in der dritten Schaltstellung den Belüftungskanal vervollständigt.

9. Anschlusseinrichtung (10) nach einem der vorhergehenden Ansprüche, **dadurch gekennzeichnet, dass** das Schließelement (18) drehbar in einem Körper (12) der Anschlusseinrichtung (10) gelagert ist, in den auch der Träger (14) eingesetzt ist.

10. Anschlusseinrichtung (10) nach Anspruch 9, **dadurch gekennzeichnet, dass** wenigstens der Körper (12) aus Edelstahl oder Aluminium gefertigt ist und fest oder lösbar mit einer Absaugvorrichtung verbunden ist.

11. Anschlusseinrichtung (10) nach Anspruch 9 oder 10, **dadurch gekennzeichnet, dass** das Schließelement (18) lösbar im Körper (12) gelagert ist.

12. Anschlusseinrichtung (10) nach einem der Ansprüche 9 bis 11, **dadurch gekennzeichnet, dass** der Belüftungskanal einen Belüftungskanalabschnitt (32) im Körper (12) und einen Belüftungskanalabschnitt (34) im Träger (14) aufweist, die über einen sich im Wesentlichen ringförmig zwischen dem Körper (12) und dem Träger (14) gebildeten, abgedichteten Hohlraum (36) in Strömungsverbindung miteinander stehen.

13. Anschlusseinrichtung (10) nach einem der vorhergehenden Ansprüche, **dadurch gekennzeichnet, dass** das Schließelement (18) ein Küken eines Absperrhahns ist.

14. Anschlusseinrichtung (10) nach einem der vorhergehenden Ansprüche, **dadurch gekennzeichnet, dass** das Schließelement (18) mechanisch an ein Bedienelement (20) gekoppelt ist.

15. Anschlusseinrichtung (10) nach einem der vorhergehenden Ansprüche, **gekennzeichnet durch** einen Rastmechanismus (22, 24), mit dem das Schließelement (18) in der jeweiligen Schaltstellung gehalten wird.

16. Anschlusseinrichtung (10) nach einem der vorhergehenden Ansprüche, **dadurch gekennzeichnet, dass** dem Belüftungskanal ein Luft-Sterilfilter (40) vorgeschaltet ist.

## Claims

1. Connection means (10) for a suction device for vacuum membrane filtration applications, comprising
a support (14) for receiving a membrane filter or a filtration base (16),
a cavity (30) formed below the membrane filter or the filtration base (16),
a suction duct which opens centrally into the cavity (30), and
a ventilation duct which opens laterally into the cavity (30),
**characterized**
**in that** the connection means (10) comprises a closing element (18) which can be moved into several switching positions and which can block or unblock both the suction duct and the ventilation duct depending on the switching positions, and
**in that** the ventilation duct is separated from the suction duct in each switching position.

2. Connection means (10) according to claim 1, **characterized in that** the ventilation duct opens into the cavity (30) via a collecting part (30a) of the cavity (30).

3. Connection means (10) according to any one of the preceding claims, **characterized in that** the closing element (18) has both at least one suction duct part (46, 50) for completing the suction duct in at least one switching position and at least one ventilation duct part (48, 52, 54) for completing the ventilation duct in at least one switching position.

4. Connection means (10) according to any one of the preceding claims, **characterized in that** in a first switching position, the suction duct is unblocked and the ventilation duct is blocked.

5. Connection means (10) according to any one of the preceding claims, **characterized in that** in a second switching position, both the suction duct and the ventilation duct are unblocked.

6. Connection means (10) according to any one of the preceding claims, **characterized in that** in a third switching position, the suction duct is blocked and the ventilation duct is unblocked.

7. Connection means (10) according to claims 4 to 6, **characterized in that** two suction duct parts (46, 50) are formed in the closing element (18), one (46) of which completes the suction duct in the first switching position and the other (50) completes the suction duct in the second switching position.

8. Connection means (10) according to claims 4 to 6 or claim 7, **characterized in that** two ventilation duct parts (52, 54) are formed in the closing element (18), one (52) of which completes the ventilation duct in the second switching position and the other (54) completes the ventilation duct in the third switching position.

9. Connection means (10) according to any one of the preceding claims, **characterized in that** the closing element (18) is rotatably mounted in a body (12) of the connection means (10), into which the support (14) is also inserted.

10. Connection means (10) according to claim 9, **characterized in that** at least the body (12) is made of stainless steel or aluminum and is firmly or detachably connected to a suction device.

11. Connection means (10) according to claim 9 or 10, **characterized in that** the closing element (18) is detachably mounted in the body (12).

12. Connection means (10) according to any one of claims 9 to 11, **characterized in that** the ventilation duct has a ventilation duct part (32) in the body (12) and a ventilation duct part (34) in the support (14), which are in flow communication with each other via a sealed cavity (36) formed substantially annularly between the body (12) and the support (14) .

13. Connection means (10) according to any one of the preceding claims, **characterized in that** the closing element (18) is a plug of a stopcock.

14. Connection means (10) according to any one of the preceding claims, **characterized in that** the closing element (18) is mechanically coupled to an operating element (20).

15. Connection means (10) according to any one of the preceding claims, **characterized by** a latching mechanism (22, 24), by means of which the closing element (18) is held in the respective switching position.

16. Connection means (10) according to any one of the preceding claims, **characterized in that** a sterile air filter (40) is connected upstream of the ventilation duct.

## Revendications

1. Système de raccordement (10) pour un dispositif d'aspiration pour des applications de filtration à membrane sous vide, comprenant
un support (14) pour la réception d'un filtre à membrane ou d'une base de filtration (16),
une cavité (30) formée sous le filtre à membrane ou la base de filtration (16),
un canal d'aspiration, qui débouche de manière centrale dans la cavité (30), et
un canal de ventilation, qui débouche latéralement dans la cavité (30),
**caractérisé en ce**
**que** le système de raccordement (10) comprend un élément de fermeture (18) mobile dans plusieurs positions de commutation, qui en fonction des positions de commutation peut bloquer ou libérer aussi bien le canal d'aspiration que le canal de ventilation, et
**que** le canal de ventilation dans chaque position de commutation est séparé du canal d'aspiration.

2. Système de raccordement (10) selon la revendication 1, **caractérisé en ce que** le canal de ventilation débouche dans la cavité (30) par l'intermédiaire d'une partie de collecte (30a) de la cavité (30).

3. Système de raccordement (10) selon l'une quelconque des revendications précédentes, **caractérisé en ce que** l'élément de fermeture (18) présente aussi bien au moins une partie de canal d'aspiration (46, 50) pour compléter le canal d'aspiration dans au moins une position de commutation qu'au moins une partie de canal de ventilation (48, 52, 54) pour compléter le canal de ventilation dans au moins une position de commutation.

4. Système de raccordement (10) selon l'une quelconque des revendications précédentes, **caractérisé en ce que** dans une première position de commutation le canal d'aspiration est libéré et le canal de ventilation est bloqué.

5. Système de raccordement (10) selon l'une quelconque des revendications précédentes, **caractérisé en ce que** dans une deuxième position de commutation aussi bien le canal d'aspiration que le canal de ventilation sont libérés.

6. Système de raccordement (10) selon l'une quelconque des revendications précédentes, **caractérisé en ce que** dans une troisième position de commutation le canal d'aspiration est bloqué et le canal de ventilation est libéré.

7. Système de raccordement (10) selon les revendications 4 à 6, **caractérisé en ce que** deux parties de canal d'aspiration (46, 50), dont l'une (46) dans la première position de commutation et l'autre (50) dans la deuxième position de commutation complète le canal d'aspiration, sont formées dans l'élément de fermeture (18).

8. Système de raccordement (10) selon les revendications 4 à 6 ou la revendication 7, **caractérisé en ce que** deux parties de canal de ventilation (52, 54), dont l'une (52) dans la deuxième position de commutation et l'autre (54) dans la troisième position de commutation complète le canal de ventilation, sont formées dans l'élément de fermeture (18).

9. Système de raccordement (10) selon l'une quelconque des revendications précédentes, **caractérisé en ce que** l'élément de fermeture (18) est monté de manière rotative dans un corps (12) du système de raccordement (10), dans lequel le support (14) est également inséré.

10. Système de raccordement (10) selon la revendication 9, **caractérisé en ce qu'**au moins le corps (12) est fabriqué à partir d'acier inoxydable ou d'aluminium et est relié fixement ou de manière détachable à un dispositif d'aspiration.

11. Système de raccordement (10) selon la revendication 9 ou 10, **caractérisé en ce que** l'élément de fermeture (18) est monté de manière détachable dans le corps (12).

12. Système de raccordement (10) selon l'une quelconque des revendications 9 à 11, **caractérisé en ce que** le canal de ventilation présente une partie de canal de ventilation (32) dans le corps (12) et une partie de canal de ventilation (34) dans le support (14), qui sont en liaison fluidique l'une avec l'autre par l'intermédiaire d'une cavité (36) étanche formée de manière sensiblement annulaire entre le corps (12) et le support (14) .

13. Système de raccordement (10) selon l'une quelconque des revendications précédentes, **caractérisé en ce que** l'élément de fermeture (18) est un boisseau d'un robinet d'arrêt.

14. Système de raccordement (10) selon l'une quelconque des revendications précédentes, **caractérisé en ce que** l'élément de fermeture (18) est accouplé mécaniquement à un élément de commande (20).

15. Système de raccordement (10) selon l'une quelconque des revendications précédentes, **caractérisé par** un mécanisme d'encliquetage (22, 24), avec lequel l'élément de fermeture (18) est maintenu dans la position de commutation respective.

16. Système de raccordement (10) selon l'une quelconque des revendications précédentes, **caractérisé en ce qu'**un filtre stérile à air (40) est monté en amont du canal de ventilation.
